(19) Europäisches Patentamt
European Patent Office
Office européen des brevets

(11) Veröffentlichungsnummer: **0 244 359 B1**

(12) **EUROPÄISCHE PATENTSCHRIFT**

(45) Veröffentlichungstag der Patentschrift: **22.07.92**

(51) Int. Cl.$^5$: **C07D 239/60**, C07D 239/56

(21) Anmeldenummer: **87810260.7**

(22) Anmeldetag: **24.04.87**

(54) **Verfahren zur Herstellung von Pyrimidinderivaten.**

(30) Priorität: **02.05.86 US 858636**

(43) Veröffentlichungstag der Anmeldung:
**04.11.87 Patentblatt 87/45**

(45) Bekanntmachung des Hinweises auf die Patenterteilung:
**22.07.92 Patentblatt 92/30**

(84) Benannte Vertragsstaaten:
**AT BE CH DE ES FR GB GR IT LI LU NL SE**

(56) Entgegenhaltungen:
**EP-A- 0 070 804**
**EP-A- 0 084 020**
**EP-A- 0 158 594**

**CHEMICAL ABSTRACTS, Band 71, Nr. 3, 21. Juli 1969, Seite 306, Spalte 1, Zusammenfassungsnr. 12881s, Columbus, Ohio, US; E. OTA et al.: "Substitution reactions of phenenthrols. VII. Reaction of 4-bromo-3-phenanthrols with anionic reagents"**

**HOUBEN-WEYL "Methoden der Organischen Chemie", 4. Auflage, Band X/1, 1971, Seiten 821-825, 882-884, Georg Thieme Verlag, Stuttgart; W. SEIDENFADEN et al.: "Methoden zur Herstellung und Umwandlung von aromatischen Nitroverbindungen"**

(73) Patentinhaber: **CIBA-GEIGY AG**
**Klybeckstrasse 141**
**CH-4002 Basel(CH)**

(72) Erfinder: **Hässig, Robert, Dr.**
**Bodenackerstrasse 222A**
**CH-4334 Sisseln(CH)**

EP 0 244 359 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von 2-Mercapto-4,6-bis-fluoralkoxypyrimidinen der Formel I

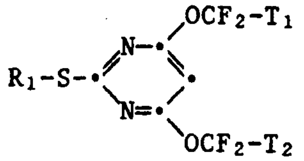

(I)

in welcher $R_1$ $C_1$-$C_4$-Alkyl, Phenyl oder Benzyl und $T_1$ und $T_2$ unabhängig voneinander je Wasserstoff, oder eine Gruppe -$CHX_1X_2$ bedeuten, in welcher $X_1$ und $X_2$ unabhängig voneinander für Trifluormethyl, Fluor, Chlor oder Brom stehen.

Die 2-Mercapto-4,6-bis-fluoralkoxypyrimidine der Formel I sind wertvolle Zwischenprodukte. Sie können beispielsweise durch Oxidation in die entsprechenden Sulfone übergeführt werden, die bei der weiteren Umsetzung mit Ammoniak oder einem primären Amin die entsprechenden 2-Amino-4,6-bis-fluoralkoxypyrimidine ergeben, die bei der weiteren Umsetzung mit einem geeigneten Phenylsulfonylisocyanat oder N-(Phenylsulfonyl)-carbamat herbizid wirksame Sulfonylharnstoffe liefern. Solche herbizid wirksamen Sulfonylharnstoffe sind beispielsweise in den publizierten europäischen Patentanmeldungen EP-A-0 072 347, EP-A-0 084 020 und EP-A-0 094 790 beschrieben.

Die 2-Mercapto-4,6-bis-fluoralkoxypyrimidine der Formel I können grundsätzlich durch direkte Umsetzung von 2-Mercapto-4,6-dihydroxypyrimidinen mit Chlordifluormethan oder eines entsprechenden 1,l-Difluoralken hergestellt werden. Nach dieses Vorfahren werden die 2-Mercapto-4,6-bis-fluoralkoxypyrimidine der Formel I jedoch nur in unbefriedigender Ausbeute erhalten. Beispielsweise wird 4,6-Bisdifluormethoxy-2-methylthiopyrimidin ausgehend von 4,6-Dihydroxy-2-methylthiopyrimidin durch Umsetzung mit Chlordifluormethan in einem aus Dioxan und wässeriger Natronlauge bestehenden Reaktionsmedium lediglich in einer Ausbeute von 25 % d. Th. erhalten (vgl. EP-A-0 158 594, Beispiel 5).

Es ist daher das Ziel der vorliegenden Erfindung, ein Verfahren zur Herstellung der 2-Mercapto-4,6-bis-fluoralkoxypyrimidine der Formel I bereitzustellen, nach dem diese Verbindungen in guter Ausbeute hergestellt werden können.

Gemäss vorliegender Erfindung wird vorgeschlagen, die 2-Mercapto-4,6-bis-fluoralkoxypyrimidine der Formel I in der Weise herzustellen, dass man ein 2-Mercapto-4-halogen-6-hydroxypyrimidin der Formel II

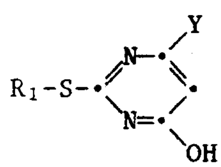

(II)

in welcher $R_1$ die unter Formel I angegebene Bedeutung hat und Y Chlor oder Brom bedeutet, mit Chlordifluormethan oder einem 1,1-Difluoralken der Formel III

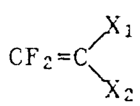

(III)

in welcher $X_1$ und $X_2$ unabhängig voneinander je Trifluormethyl, Fluor, Chlor oder Brom bedeuten, in einem inerten Lösungsmittel in Gegenwart einer starken Base zu einem 2-Mercapto-4-halogen-6-fluoralkoxypyrimidin der Formel IV

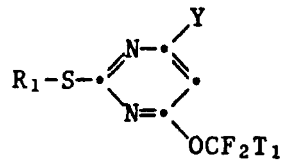

(IV)

in welcher $R_1$, Y und $T_1$ die oben angegebene Bedeutung haben, umsetzt, dieses durch weitere Umsetzung mit einem Alkali- oder Erdalkalimetallnitrit in einem polaren Lösungsmittel in ein 2-Mercapto-4-hydroxy-6-fluoralkoxypyrimidin der Formel V

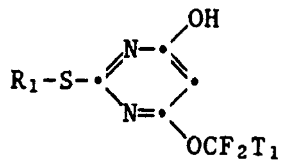

(V)

worin $R_1$ und $T_1$ die oben angegebene Bedeutung haben, umwandelt und dieses dann durch Umsetzung mit Chlordifluormethan oder einem 1,1-Difluoralken der Formel III in einem inerten Lösungmittel in Gegenwart einer starken Base in ein 2-Mercapto-4,6-bis-fluoralkoxypyrimidin der Formel I überführt.

Bevorzugte 2-Mercapto-4,6-bis-fluoralkoxypyrimidine der Formel I, die nach dem erfindungsgemässen Verfahren hergestellt werden können, sind solche, in welchen $R_1$ $C_1$-$C_2$-Alkyl oder Benzyl, $T_1$ Wasserstoff und $T_2$ eine Gruppe-$CHFX_1$ bedeutet. Besonders bevorzugt sind 2-Mercapto-4,6-bis-fluoralkoxypyrimidine der Formel I, in welchen $R_1$ $C_1$-$C_2$-Alkyl und $T_1$ und $T_2$ Wasserstoff sind.

Die 2-Mercapto-4-halogen-6-hydroxypyrimidine der Formel II sind bekannte Verbindungen, die in bekannter Weise durch Umsetzung von 2-Mercapto-4,6-dihydroxypyrimidinen mit einem anorganischen Säurehalogenid, wie Thionylchlorid, Phosgen, Phosphoroxychlorid oder Phosphorpentachlorid, zu den entsprechenden 2-Mercapto-4,6-dihalogenpyrimidinenund nachfolgende partielle Hydrolyse in guter Ausbeute hergestellt werden können.

Die Umsetzung der 2-Mercapto-4-halogen-6-hydroxypyrimidine der Formel II mit Chlordifluormethan oder einem 1,1-Difluoralken der Formel III wird vorteilhaft in einem inerten Lösungsmittel vorgenommen. Besonders geeignet sind polare Lösungsmittel, wie flüssige Alkancarbonsäureamide, Nitrile, Dialkylsulfoxide, Aether, Ketone und Alkohole. Ferner können tertiäre Amine als Lösungsmittel dienen. Im einzelnen sind als geeignete Lösungsmittel beispielsweise Formamid, N,N-Dimethylformamid, N,N-Dimethylacetamid, Acetonitril, Dimethylsulfoxid, Diäthyläther, Tetrahydrofuran, Dioxan, Aceton, Methyläthylketon, Methylisobutylketon, und $C_1$-$C_4$-Alkanole, wie Methanol, Ethanol und Isopropanol, zu nennen. Als tertiäre Amine kommen insbesondere Triäthylamin, Pyridin, Picoline, oder N,N-Dialkylaniline, insbesondere N,N-Dimethylanilin, in Betracht. Ein bevorzugtes Lösungsmittel ist Dioxan.

Als starke Basen, in deren Gegenwart die Umsetzung eines 2-Mercapto-4-halogen-6-hydroxypyrimidins der Formel II mit Chlordifluormethan oder einem 1,1-Difluoralken der Formel III durchgeführt wird, kommen Alkali- und Erdalkalimetallhydroxide und tertiäre organische Basen in Betracht. Geeignete Basen sind beispielsweise Lithiumhydroxid, Natriumhydroxid, Kaliumhydroxid, Calciumhydroxid, Strontiumhydroxid, Bariumhydroxid, Triäthylamin, N,N-Dimethylanilin und Pyridin. Bevorzugte Basen sind Natriumhydroxid, Kaliumhydroxid und Calciumhydroxid. Besonders bevorzugt sind Natriumhydroxid und Kaliumhydroxid. Die Basen werden in mindestens stöchiometrischer Menge eingesetzt. In der Regel werden die Basen jedoch im Ueberschuss eingesetzt. Beispielsweise hat sich die Verwendung von 5-10 Aequivalenten Base pro Mol 2-Mercapto-4-halogen-6-hydroxypyrimidin der Formel II in der Praxis bewährt. Die Basen können vorteilhaft in Form ihrer wässerigen Lösungen, beispielsweise in Form von 50%iger wässriger Natronlauge oder von 50%iger wässriger Kalilauge, eingesetzt werden.

Die Umsetzung eines 2-Mercapto-4-halogen-6-hydroxypyrimidins der Formel II mit Chlordifluormethan oder einem 1,1-Difluor-2,2-dihalogenäthylen der Formel III wird vorteilhaft in Gegenwart eines Phasentransferkatalysators vorgenommen. Geeignete Phasentransferkatalysatoren sind insbesondere Kronenäther und quaternäre Ammoniumsalze. Als besonders geeignete Phasentransferkatalysatoren sind 18-Crown-6, Tetrabutylammoniumbromid und Benzyltriäthylammoniumchlorid zu nennen. Die Phasentransferkatalysatoren werden in der Regel in einer Menge von 1-20 Mol% bezogen auf 2-Mercapto-4-halogen-6-hydroxypyrimidin der Formel II eingesetzt. Vorzugsweise verwendet man 2,5-5 Mol% Phasentransferkatalysator pro Mol 2-Mercapto-4-halogen-6-hydroxypyrimidin der Formel II.

Der Druck, bei dem die Umsetzung eines 2-Mercapto-4-halogen-6-hydroxypyrimidins der Formel II mit Chlordifluormethan oder einem 1,1-Difluoralken der Formel III durchgeführt wird, kann in weiten Grenzen variieren. Geeignete Drucke liegen im Bereich von 0,1-20 bar. Vorzugsweise wird die Umsetzung bei Normaldruck oder mässig erhöhtem Druck durchgeführt. Ein bevorzugter Druckbereich, in dem die Reaktion durchgeführt werden kann, liegt bei 0,8-5 bar.

Als polare Lösungsmittel, in deren Gegenwart die Umsetzung eines 2-Mercapto-4-halogen-6-fluoralkoxypyrimidins der Formel IV mit einem Alkali- oder Erdalkalimetallnitrit durchgeführt wird, kommen flüssige Alkancarbonsäureamide und -nitrile, Lactame, Dialkylsulfoxide und $C_1$-$C_4$-Alkanole in Betracht. Im einzelnen sind Formamid, N,N-Dimethylformamid, N,N-Dimethylacetamid, N-Methylpyrrolidon, Dimethylsulfoxid, Methanol, Aethanol und Isopropanol als geeignete Lösungs-mittel zu nennen. Bevorzugte Lösungsmittel, in deren Gegenwart die Umsetzung eines 2-Mercapto-4-halogen-6-fluoralkoxypyrimidins der Formel IV mit einem Alkali- oder einem Erdalkalimetallnitrit durchgeführt werden kann, sind Formamid, N,N-Dimethylformamid und Dimethylsulfoxid. Ein besonders bevorzugtes Lösungsmittel ist N,N-Dimethylformamid.

Als Alkali- und Erdalkalimetallnitrite kommen beispielsweise Natriumnitrit, Kaliumnitrit, Ammoniumnitrit, Magnesiumnitrit, Calciumnitrit und Bariumnitrit in Betracht. Bevorzugte Nitrite sind Natriumnitrit und Kaliumnitrit. Die Nitrite werden in mindestens stöchiometrischer Menge bezogen auf das 2-Mercapto-4-halogen-6-fluoralkoxypyrimidin der Formel IV verwendet. In der Regel verwendet man jedoch einen Ueberschuss von 2-5 Mol Nitrit pro Mol 2-Mercapto-4-halogen-6-fluoralkoxypyrimidin der Formel IV. Vorzugsweise verwendet man 2-4 Mol Nitrit pro Mol 2-Mercapto-4-halogen-6-fluoralkoxypyrimidin der Formel IV.

Die Umsetzung eines 2-Mercapto-4-halogen-6-fluoralkoxypyrimidins der Formel IV mit einem Alkali- oder Erdalkalimetallnitrit wird vorteilhaft bei erhöhter Temperatur durchgeführt. Geeignete Reaktionstemperaturen liegen im Bereich von 50-200 °C. Vorzugsweise wird die Umsetzung bei einer Temperatur von 130-160 °C durchgeführt.

Die Umsetzung eines 2-Mercapto-4-hydroxy-6-fluoralkoxypyrimidins der Formel V mit Chlordifluormethan oder einem 1,1-Difluoralken der Formel III entspricht im wesentlichen der oben beschriebenen Umsetzung eines 2-Mercapto-4-halogen-6-hydroxypyrimidins der Formel II mit Chlordifluormethan oder einem 1,1-Difluor-2,2-dihalogenäthylen der Formel III. Daher gelten die bezüglich verwendbarer Lösungsmittel, Reaktionstemperaturen und Katalysatoren bereits im Zusammenhang mit der Umsetzung von 2-Mercapto-4-halogen-6-hydroxypyrimidin in der Formel II mit Chlordifluormethan oder einem 1,1-Difluoralken der Formel III gemachten Ausführungen für diese Umsetzung in analoger Weise.

Die durch Umsetzung eines 2-Mercapto-4-halogen-6-hydroxypyrimidins der Formel II mit Chlordifluormethan oder einem 1,1-Difluoralken der Formel III erhältlichen 2-Mercapto-4-halogen-6-fluoralkoxypyrimidine der Formel IV sind neue Verbindungen und ebenfalls Gegenstand der Erfindung.

Mit dem erfindungsgemässen Verfahren wird es möglich, die 2-Mercapto-4,6-bis-fluoralkoxypyrimidine der Formel I ausgehend von den leicht zugänglichen 2-Mercapto-4-halogen-6-hydroxypyrimidinen der Formel II in Ausbeuten von über 50% d. Th. herzustellen. Da die als Ausgangsmaterial verwendeten 2-Mercapto-4-halogen-6-hydroxypyrimidine der Formel II ihrerseits ausgehend von den entsprechenden 2-Mercapto-4,6-dihydroxypyrimidinen in Ausbeuten von über 80 % d. Th. herstellbar sind, beträgt die mit dem erfindungsgemässen Verfahren erzielbare Ausbeute an 2-Mercapto-4,6-bis-fluoralkoxypyrimidinen der Formel I bezogen auf die entsprechenden 4,6-Dihydroxypyrimidine über 40 % d. Th., während, wie eingangs erwähnt, die direkte Umsetzung der 4,6-Dihydroxypyrimidine mit Chlordifluormethan die 4,6-bis-Difluormethoxy-Verbindungen lediglich in einer Ausbeute von 25 % d. Th. liefert.

Das erfindungsgemässe Verfahren wird durch die folgenden Beispiele näher erläutert.

Beispiel 1: Herstellung von 4-Chlor-6-difluormethoxy-2-methylthiopyrimidin.

6,8 g (0,036 Mol) 4-Chlor-6-hydroxy-2-methylthiopyrimidin werden in 25 ml 50%iger Kalilauge, 25 ml Wasser und 100 ml Dioxan suspendiert. Nach Zugabe von 0,5 g 18-Crown-6 wird das Reaktionsgemisch auf 40 °C erwärmt und anschliessend während 3 Stunden 10 g Chlordifluormethan eingeleitet. Danach wird die Mischung auf Raumtemperatur abgekühlt, die Phasen getrennt und die organische Phase zur Trockene eingedampft. Der Rückstand wird aus Methanol/Wasser kristallisiert. Es werden 6,9 g (84,4 % d. Th.) 4-Chlor-6-difluormethoxy-2-methylthiopyrimidinvom Schmelzpunkt 36-37 °C erhalten. Die Substanz hat einen Siedepunkt von 76-78 °C bei 0,15 mbar.

Beispiel 2: Herstellung von 4-Difluormethoxy-6-hydroxy-2-methylthio-1,3-pyrimidin.

In eine Lösung von 8,7 g (0,038 Mol) 4-Chlor-6-difluormethoxy-2-methylthiopyrimidin in 10 ml Dimethylformamid wird bei 140 °C während 50 Min. eine Lösung von 8,8 g (0,127 Mol) Natriumnitrit in 10 ml

Dimethylformamid eingetropft. Nach beendigter Zugabe des Natriumnitrits wird 2 Stunden bei 140-150 ° C nachgerührt. Dann wird das Lösungsmittel im Vakuum abgedampft und der Rückstand aus 1N Salzsäure kristallisiert. Es werden 5,6 g (70 % d. Th.) 4-Difluormethoxy-6-hydroxy-2-methylthiopyrimidin vom Schmelzpunkt 236-237 ° C erhalten.

Beispiel 3: Herstellung von 4,6-Bis-difluormethoxy-2-methylthiopyrimidin.

14,0 g (0,067 Mol) 4-Difluormethoxy-6-hydroxy-2-methylthiopyrimidin werden in 18 ml 50%iger Kalilauge, 10 ml Wasser und 160 ml Dioxan suspendiert. Nach Zugabe von 0,5 g 18-Crown-6 wird das Reaktionsgemisch auf 40 ° C erwärmt und während 5 Stunden 20 g Chlordifluormethan eingeleitet. Danach wird die wässerige Phase abgetrennt und die organische Phase im Hochvakuum destilliert. Es werden 15,0 g (86,4 % d. Th.) 4,6-Bis-difluormethoxy-2-methylthiopyrimidin vom Siedepunkt 63 ° C/0,06 mbar erhalten. Die Substanz schmilzt bei 46-48 ° C.

Beispiel 4: Herstellung von 4-Difluormethoxy-6-(1,1,2,3,3,3-hexafluorpropoxy)-2-methylthiopyrimidin.

In eine Lösung von 20,8 g (0,1 Mol) 4-Difluormethoxy-6-hydroxy-2-methylthiopyrimidin und 1,0 g Triäthylamin in 80 ml Dimethylformamid werden unter Rühren bei 20-25 ° C während 6 Stunden 21,0 g (0,14 Mol) Hexafluorpropylen eingeleitet. Nach beendigter Zugabe des Hexafluorpropylens wird das Reaktionsgemisch noch 30 Minuten gerührt und dann über Hyflo filtriert. Das klare Filtrat wird bei einer Badtemperatur von 70 ° C und einem Druck von 33 mbar von Lösungsmittel befreit. Als Rückstand werden 31,2 g (87 % der Theorie) Rohprodukt erhalten, das durch Destillation im Hochvakuum gereinigt wird. Ausbeute: 25,2 g (70,4 % der Theorie); Siedepunkt: 88 ° C/0,33 mbar.

In analoger Weise wie in Beispielen 1-3 beschrieben, werden folgende 2-Mercapto-4,6-bis-fluoralkoxypyrimidine der Formel I erhalten:

| $R_1$ | $T_1$ | $T_2$ | |
|---|---|---|---|
| $CH_3$ | H | CHClF | Sdp.: 117°C/0,07 mbar |
| $C_2H_5$ | H | H | Sdp.: 85°C/0,05 mbar |
| 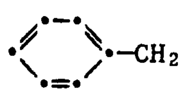 | H | H | Smp.: 40-43°C |
| $C_2H_5$ | H | CHClF | |

## Patentansprüche

**1.** Verfahren zur Herstellung von 2-Mercapto-4,6-bis-fluoralkoxypyrimidinen der Formel I

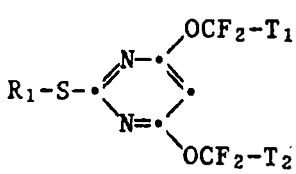

(I)

in welcher $R_1$ $C_1$-$C_4$-Alkyl, Phenyl oder Benzyl und $T_1$ und $T_2$ unabhängig voneinander je Wasserstoff, oder eine Gruppe-$CHX_1X_2$ bedeuten, in welcher $X_1$ und $X_2$ unabhängig voneinander für Trifluormethyl, Fluor, Chlor oder Brom stehen, dadurch gekennzeichnet, dass man ein 2-Mercapto-4-halogen-6-hydroxypyrimidin der Formel II

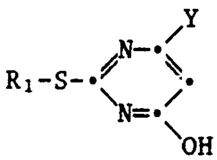

(II)

in welcher $R_1$ die unter Formel I angegebene Bedeutung hat und Y Chlor oder Brom bedeutet, mit Chlordifluormethan oder einem 1,1-Difluoralken der Formel III

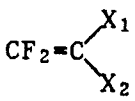

(III)

in welcher $X_1$ und $X_2$ unabhängig voneinander je Trifluormethyl, Fluor, Chlor oder Brom bedeuten, in einem inerten Lösungsmittel in Gegenwart einer starken Base zu einem 2-Mercapto-4-halogen-6-fluoralkoxypyrimidin der Formel IV

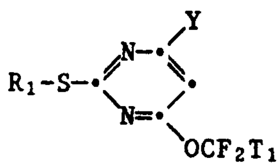

(IV)

in welcher $R_1$, Y und $T_1$ die oben angegebene Bedeutung haben, umsetzt, dieses durch weitere Umsetzung mit einem Alkali- oder Erdalkalimetallnitrit in einem polaren Lösungsmittel in ein 2-Mercapto-4-hydroxy-6-fluoralkoxypyrimidin der Formel V

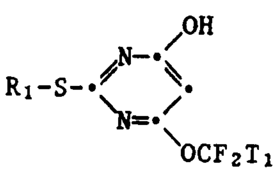

(V)

worin $R_1$ und $T_1$ die oben angegebene Bedeutung haben, umwandelt und dieses dann durch Umsetzung mit Chlordifluormethan oder einem 1,1-Difluoralken der Formel III in einem inerten Lösungmittel in Gegenwart einer starken Base in ein 2-Mercapto-4,6-bis-fluoralkoxypyrimidin der Formel I überführt.

2. Verfahren nach Anspruch 1, dadurch gekennzeichnet, dass man die Umsetzung eines 2-Mercapto-4-halogen-6-hydroxypyrimidins der Formel II oder eines 2-Mercapto-4-hydroxy-6-fluoralkoxypyrimidins der Formel V mit Chlordifluormethan oder einem 1,1-Difluoralken der Formel III in einem flüssigen Alkancarbonsäureamid, einem Alkancarbonsäurenitril, einem Dialkylsulfoxid, einem Aether, einem Keton oder einem Alkohol als Lösungsmittel durchführt.

3. Verfahren nach Anspruch 2, dadurch gekennzeichnet, dass man die Umsetzung eines 2-Mercapto-4-halogen-6-hydroxypyrimidins der Formel II oder eines 2-Mercapto-4-hydroxy-6-fluoralkoxypyrimidins der Formel V mit Chlordifluormethan oder einem 1,1-Difluoralken der Formel III in Formamid, N,N-Dimethylformamid, N,N-Dimethylacetamid, Acetonitril, Dimethylsulfoxid, Diäthyläther, Tetrahydrofuran, Dioxan, Aceton, Methyläthylketon, Methylisobutylketon oder einem $C_1$-$C_4$-Alkanol als Lösungsmittel durchführt.

4. Verfahren nach Anspruch 2, dadurch gekennzeichnet, dass man die Umsetzung eines 2-Mercapto-4-halogen-6-hydroxypyrimidins der Formel II oder eines 2-Mercapto-4-hydroxy-6-fluoralkoxypyrimidins der Formel V mit Chlordifluormethan oder einem 1,1-Difluoralken der Formel III in Dioxan als Lösungsmittel durchführt.

**5.** Verfahren nach Anspruch 1, dadurch gekennzeichnet, dass man die Umsetzung eines 2-Mercapto-4-halogen-6-hydroxypyrimidins der Formel II oder eines 2-Mercapto-4-hydroxy-6-fluorakoxypyrimidins der Formel V mit Chlordifluormethan oder einem 1,1-Difluoralken der Formel III in Gegenwart eines Alkali- oder Erdalkalimetallhydroxids durchführt.

**6.** Verfahren nach Anspruch 5, dadurch gekennzeichnet, dass man die Umsetzung eines 2-Mercapto-4-halogen-6-hydroxypyrimidins der Formel II oder eines 2-Mercapto-4-hydroxy-6-fluoralkoxypyrimidins der Formel V mit Chlordifluormethan oder einem 1,1-Difluoralken der Formel III in Gegenwart von Lithiumhydroxid, Natriumhydroxid, Kaliumhydroxid, Calciumhydroxid, Strontiumhydroxid oder Bariumhydroxid durchführt.

**7.** Verfahren nach Anspruch 6, dadurch gekennzeichnet, dass man die Umsetzung eines 2-Mercapto-4-halogen-6-hydroxypyrimidins der Formel II oder eines 2-Mercapto-4-hydroxy-6-fluoralkoxypyrimidins der Formel V mit Chlordifluormethan oder einem 1,1-Difluoralken der Formel III in Gegenwart von Natriumhydroxid oder Kaliumhydroxid durchführt.

**8.** Verfahren nach Anspruch 1, dadurch gekennzeichnet, dass man die Basen in Form ihrer wässerigen Lösungen einsetzt.

**9.** Verfahren nach Anspruch 1, dadurch gekennzeichnet, dass man die Umsetzung eines 2-Mercapto-4-halogen-6-hydroxypyrimidins der Formel II oder eines 2-Mercapto-4-hydroxy-6-fluoralkoxypyrimidins der Formel V mit Chlordifluormethan oder einem 1,1-Difluoralken der Formel III in Gegenwart eines Phasentransferkatalysators durchführt.

**10.** Verfahren nach Anspruch 9, dadurch gekennzeichnet, dass man den Phasentransferkatalysator in einer Menge von 1 - 20 Mol% bezogen auf das 2-Mercapto-4-halogen-6-hydroxypyrimidin der Formel II oder das 2-Mercapto-4-hydroxy-6-fluoralkoxypyrimidin der Formel V einsetzt.

**11.** Verfahren nach Ansprüchen 9 und 10, dadurch gekennzeichnet, dass man als Phasentransferkatalysator einen Kronenäther oder ein quaternäres Ammoniumsalz verwendet.

**12.** Verfahren nach Ansprüchen 9 und 10, dadurch gekennzeichnet, dass man als Phasentransferkatalysator 18-Crown-6, Tetrabutylammoniumbromid oder Benzyltriäthylammoniumchlorid verwendet.

**13.** Verfahren nach Anspruch 1, dadurch gekennzeichnet, dass man die Umsetzung eines 2-Mercapto-4-halogen-6-fluoralkoxypyrimidins der Formel IV mit einem Alkali- oder Erdalkalimetallnitrit in einem flüssigen Alkancarbonsäureamid, einem flüssigen Alkancarbonsäurenitril, einem Lactam, einem Dialkylsulfoxid oder einem $C_1$-$C_4$-Alkanol durchführt.

**14.** Verfahren nach Anspruch 13, dadurch gekennzeichnet, dass man die Umsetzung eines 2-Mercapto-4-halogen-6-fluoralkoxypyrimidins der Formel IV mit einem Alkali- oder Erdalkalimetallnitrit in Formamid, N,N-Dimethylformamid, N,N-Dimethylacetamid, N-Methylpyrolidon, Dimethylsulfoxid, Methanol, Aethanol oder Isopropanol als Lösungsmittel durchführt.

**15.** Verfahren nach Anspruch 14, dadurch gekennzeichnet, dass man die Umsetzung eines 2-Mercapto-4-halogen-6-fluoralkoxypyrimidins der Formel IV mit einem Alkali- oder Erdalkalimetallnitrit in Formamid, N,N-Dimethylformamid oder Dimethylsulfoxid als Lösungsmittel durchführt.

**16.** Verfahren nach Anspruch 15, dadurch gekennzeichnet, dass man die Umsetzung eines 2-Mercapto-4-halogen-6-fluoralkoxypyrimidins der Formel IV mit einem Alkali- oder Erdalkalimetallnitril in N,N-Dimethylformamid als Lösungsmittel durchführt.

**17.** Verfahren nach Anspruch 1, dadurch gekennzeichnet, dass man als Alkali- oder Erdalkalimetallnitrit, Natriumnitrit, Kaliumnitrit, Ammoniumnitrit, Magnesiumnitrit, Calciumnitrit oder Bariumnitrit verwendet.

**18.** Verfahren nach Anspruch 17, dadurch gekennzeichnet, dass man Natriumnitrit oder Kaliumnitrit verwendet.

**19.** Verfahren nach Anspruch 1, dadurch gekennzeichnet, dass man 2-4 Mol Alkali- oder Erdalkalimetallnitrit pro Mol 2-Mercapto-4-halogen-6-fluoralkoxypyrimidin der Formel IV verwendet.

**20.** Verfahren nach Anspruch 1, dadurch gekennzeichnet, dass man die Umsetzung eines 2-Mercapto-4-halogen-6-fluoralkoxypyrimidins der Formel IV mit einem Alkali- oder Erdalkalimetallnitrit bei einer Temperatur im Bereich von 50-200 ° C durchführt.

**21.** Verfahren nach Anspruch 20, dadurch gekennzeichnet, dass man die Umsetzung eines 2-Mercapto-4-halogen-6-fluoralkoxypyrimidins der Formel IV mit einem Alkali- oder Erdalkalimetallnitrit bei einer Temperatur von 130-160 ° C durchführt.

**22.** 2-Mercapto-4-halogen-6-fluoralkoxypyrimidine der Formel IV

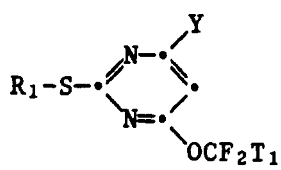

(IV)

in welcher $R_1$ $C_1$-$C_4$-Alkyl, Phenyl oder Benzyl, Y Chlor oder Brom und $T_1$ Wasserstoff oder eine Gruppe -$CHX_1X_2$ bedeutet, in welcher $X_1$ und $X_2$ unabhängig voneinander für Trifluormethyl, Fluor, Chlor oder Brom stehen.

**Claims**

**1.** A process for the preparation of a 2-mercapto-4,6-bisfluoroalkoxypyrimidine of the formula I

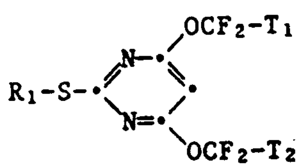

(I)

in which $R_1$ is $C_1$-$C_4$-alkyl, phenyl or benzyl and each of $T_1$ and $T_2$ independently of the other is hydrogen or a group -$CHX_1X_2$ in which $X_1$ and $X_2$ independently of the other are trifluoromethyl, fluorine, chlorine or bromine, which comprises reacting a 2-mercapto-4-halo-6-hydroxypyrimidine of the formula II

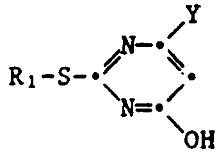

(II)

in which $R_1$ is as defined for formula I and Y is chlorine or bromine, with chlorodifluoromethane or a 1,1-difluoroalkene of the formula III

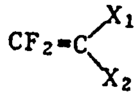

(III)

in which each of $X_1$ and $X_2$ independently of the other is trifluoromethyl, fluorine, chlorine or bromine, in an inert solvent in the presence of a strong base, to give a 2-mercapto-4-halo-6-fluoroalkoxypyrimidine of formula IV

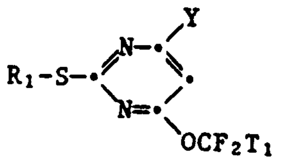

(IV)

in which $R_1$, Y and $T_1$ are as defined above, converting the latter compound by further reaction with an alkali metal nitrite or an alkaline earth metal nitrite, in a polar solvent, into a 2-mercapto-4-hydroxy-6-fluoroalkoxypyrimidine of formula V

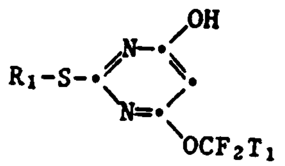

(V)

in which $R_1$ and $T_1$ are as defined above, and then converting the latter compound by reaction with chlorodifluoromethane or a 1,1-difluoroalkene of formula III, in an inert solvent in the presence of a strong base, into a 2-mercapto-4,6-bisfluoroalkoxypyrimidine of formula I.

**2.** A process according to claim 1, wherein the reaction of a 2-mercapto-4-halo-6-hydroxypyrimidine of formula II or of a 2-mercapto-4-hydroxy-6-fluoroalkoxypyrimidine of formula V with chlorodifluoromethane or a 1,1-difluoroalkene of formula III is carried out in a liquid alkanecarboxamide, an alkanecarboxylic acid nitrile, a dialkyl sulfoxide, an ether, a ketone or an alcohol as solvent.

**3.** A process according to claim 2, wherein the reaction of a 2-mercapto-4-halo-6-hydroxypyrimidine of formula II or of a 2-mercapto-4-hydroxy-6-fluoroalkoxypyrimidine of formula V with chlorodifluoromethane or a 1,1-difluoroalkene of formula III is carried out in formamide, N,N-dimethylformamide, N,N-dimethylacetamide, acetonitrile, dimethyl sulfoxide, diethyl ether, tetrahydrofuran, dioxane, acetone, methyl ethyl ketone, methyl isobutyl ketone or a $C_1$-$C_4$ alkanol as solvent.

**4.** A process according to claim 2, wherein the reaction of a 2-mercapto-4-halo-6-hydroxypyrimidine of formula II or of a 2-mercapto-4-hydroxy-6-fluoroalkoxypyrimidine of formula V with chlorodifluoromethane or a 1,1-difluoroalkene of formula III is carried out in dioxane as solvent.

**5.** A process according to claim 1, wherein the reaction of a 2-mercapto-4-halo-6-hydroxypyrimidine of formula II or of a 2-mercapto-4-hydroxy-6-fluoroalkoxypyrimidine of formula V with chlorodifluoromethane or a 1,1-difluoroalkene of formula III is carried out in the presence of an alkali metal hydroxide or an alkaline earth metal hydroxide.

**6.** A process according to claim 5, wherein the reaction of a 2-mercapto-4-halo-6-hydroxypyrimidine of formula II or of a 2-mercapto-4-hydroxy-6-fluoroalkoxypyrimidine of formula V with chlorodifluoromethane or a 1,1-difluoroalkene of formula III is carried out in the presence of lithium hydroxide, sodium hydroxide, potassium hydroxide, calcium hydroxide, strontium hydroxide or barium hydroxide.

**7.** A process according to claim 6, wherein the reaction of a 2-mercapto-4-halo-6-hydroxypyrimidine of formula II or of a 2-mercapto-4-hydroxy-6-fluoroalkoxypyrimidine of formula V with chlorodifluoromethane or a 1,1-difluoroalkene of formula III is carried out in the presence of sodium hydroxide or potassium hydroxide.

**8.** A process according to claim 1, wherein the bases are employed in the form of their aqueous solutions.

**9.** A process according to claim 1, wherein the reaction of a 2-mercapto-4-halo-6-hydroxypyrimidine of formula II or of a 2-mercapto-4-hydroxy-6-fluoroalkoxypyrimidine of formula V with chlorodifluoromethane or a 1,1-difluoroalkene of formula III is carried out in the presence of a phase transfer catalyst.

**10.** A process according to claim 9, wherein the phase transfer catalyst is employed in an amount of 1 to 20 mol%, based on the 2-mercapto-4-halo-6-hydroxypyrimidine of formula II or the 2-mercapto-4-hydroxy-6-fluoroalkoxypyrimidine of formula V.

**11.** A process according to claims 9 and 10, wherein a crown ether or a quaternary ammonium salt is used as phase transfer catalyst.

**12.** A process according to claims 9 and 10, wherein 18-crown-6, tetrabutylammonium bromide or benzyltriethylammonium chloride is used as phase transfer catalyst.

**13.** A process according to claim 1, wherein the reaction of a 2-mercapto-4-halo-6-fluoroalkoxypyrimidine of formula IV with an alkali metal nitrite or an alkaline earth metal nitrite is carried out in a liquid alkanecarboxamide, a liquid alkanecarboxylic acid nitrile, a lactam, a dialkyl sulfoxide or a $C_1$-$C_4$-alkanol.

**14.** A process according to claim 13, wherein the reaction of a 2-mercapto-4-halo-6-fluoroalkoxypyrimidine of formula IV with an alkali metal nitrite or an alkaline earth metal nitrite is carried out in formamide, N,N-dimethylformamide, N,N-dimethylacetamide, N-methylpyrrolidone, dimethyl sulfoxide, methanol, ethanol or isopropanol as solvent.

**15.** A process according to claim 14, wherein the reaction of a 2-mercapto-4-halo-6-fluoroalkoxypyrimidine of formula IV with an alkali metal nitrite or an alkaline earth metal nitrite is carried out in formamide, N,N-dimethylformamide or dimethyl sulfoxide as solvent.

**16.** A process according to claim 15, wherein the reaction of a 2-mercapto-4-halo-6-fluoroalkoxypyrimidine of formula IV with an alkali metal nitrite or an alkaline earth metal nitrite is carried out in N,N-dimethylformamide as solvent.

**17.** A process according to claim 1, wherein sodium nitrite, potassium nitrite, ammonium nitrite, magnesium nitrite, calcium nitrite or barium nitrite is used as alkali metal nitrite or alkaline earth metal nitrite.

**18.** A process according to claim 17, which comprises the use of sodium nitrite or potassium nitrite.

**19.** A process according to claim 1, wherein 2 to 4 moles of alkali metal nitrite or alkaline earth metal nitrite are used per mole of 2-mercapto-4-halo-6-fluoroalkoxypyrimidine of formula IV.

**20.** A process according to claim 1, wherein the reaction of a 2-mercapto-4-halo-6-fluoroalkoxypyrimidine of formula IV with an alkali metal nitrite or an alkaline earth metal nitrite is carried out at a temperature in the range from 50 to 200 ° C.

**21.** A process according to claim 20, wherein the reaction of a 2-mercapto-4-halo-6-fluoroalkoxypyrimidine of formula IV with an alkali metal nitrite or an alkaline earth metal nitrite is carried out at a temperature from 130 to 160 ° C.

**22.** A 2-mercapto-4-halo-6-fluoroalkoxypyrimidine of formula IV

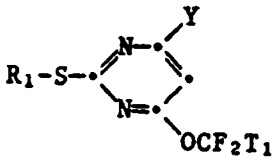

(IV)

in which $R_1$ is $C_1$-$C_4$-alkyl, phenyl or benzyl, Y is chlorine or bromine and $T_1$ is hydrogen or a group $-CHX_1X_2$ in which $X_1$ and $X_2$ independently of the other are trifluoromethyl, fluorine, chlorine or bromine.

## Revendications

**1.** Procédé de préparation des 2-mercapto-4,6-bis-fluoralcoxypyrimidines de formule I

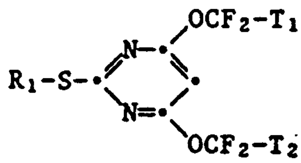

(I)

dans laquelle $R_1$ représente un groupe alkyle en C1-C4, phényle ou benzyle et $T_1$ et $T_2$ représentent chacun, indépendamment l'un de l'autre, l'hydrogène ou un groupe $-CHX_1X_2$ dans lequel $X_1$ et $X_2$ représentent chacun, indépendamment l'un de l'autre, un groupe trifluorométhyle, le fluor, le chlore ou le brome, caractérisé en ce que l'on fait réagir dans un solvant inerte en présence d'une base forte une 2-mercapto-4-halogéno-6-hydroxypyrimidine de formule II

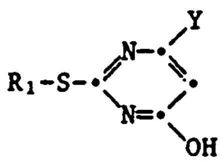

(II)

dans laquelle $R_1$ a les significations indiquées en référence à la formule I et Y représente le chlore ou le brome, avec le chlorodifluorométhane ou un 1,1-difluoralcène de formule III

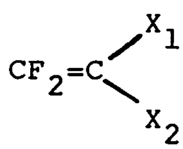

(III)

dans laquelle $X_1$ et $X_2$ représentent chacun, indépendamment l'un de l'autre, un groupe trifluorométhyle, le fluor, le chlore ou le brome, la réaction donnant une 2-mercapto-4-halogéno-6-fluoralcoxypyrimidine de formule IV

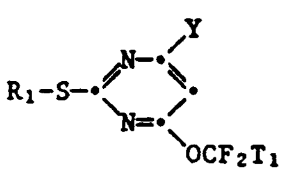

(IV)

dans laquelle $R_1$, Y et $T_1$ ont les significations indiquées ci-dessus, qu'on convertit par réaction avec un nitrite de métal alcalin ou alcalino-terreux dans un solvant polaire en une 2-mercapto-4-hydroxy-6-fluoralcoxypyrimidine de formule V

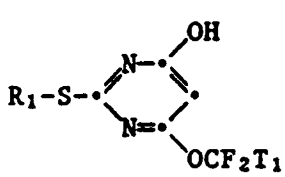

(V)

dans laquelle $R_1$ et $T_1$ ont les significations indiquées ci-dessus, qu'on convertit elle-même, par réaction avec le chlorodifluorométhane ou un 1,1-difluoralcène de formule III dans un solvant inerte en présence d'une base forte, en une 2-mercapto-4,6-bis-fluoralcoxypyrimidine de formule I.

**2.** Procédé selon la revendication 1, caractérisé en ce que la réaction entre une 2-mercapto-4-halogéno-6-hydroxypyrimidinede formule II ou une 2-mercapto-4-hydroxy-6-fluoralcoxypyrimidine de formule V et le chlorodifluorométhane ou un 1,1-difluoralcène de formule III est effectuée dans un alcane-carboxamide liquide, un alcane-carboxynitrile liquide, un dialkylsulfoxyde liquide, un éther, une cétone ou un alcool liquides, servant de solvants.

**3.** Procédé selon la revendication 2, caractérisé en ce que la réaction entre une 2-mercapto-4-halogéno-6-hydroxypyrimidinede formule II ou une 2-mercapto-4-hydroxy-6-fluoralcoxypyrimidine de formule V et le chlorodifluorométhane ou un 1,1-difluoralcène de formule III est réalisée dans le formamide, le N,N-diméthylformamide, le N,N-diméthylacétamide, l'acétonitrile, le diméthylsulfoxyde, l'éther éthylique, le tétrahydrofuranne, le dioxanne, l'acétone, la méthyléthylcétone, la méthylisobutylcétone ou un alcanol en C1-C4, servant de solvant.

**4.** Procédé selon la revendication 2, caractérisé en ce que la réaction entre une 2-mercapto-4-halogéno-6-hydroxypyrimidinede formule II ou une 2-mercapto-4-hydroxy-6-fluoralcoxypyrimidine de formule V et le chlorodifluorométhane ou un 1,1-difluoralcène de formule III est réalisée dans le dioxanne servant de solvant.

**5.** Procédé selon la revendication 1, caractérisé en ce que la réaction entre une 2-mercapto-4-halogéno-6-hydroxypyrimidinede formule II ou une 2-mercapto-4-hydroxy-6-fluoralcoxypyrimidine de formule V et le chlorodifluorométhane ou un 1,1-difluoralcène de formule III est réalisée en présence d'un hydroxyde de métal alcalin ou alcalino-terreux.

**6.** Procédé selon la revendication 5, caractérisé en ce que la réaction entre une 2-mercapto-4-halogéno-6-hydroxypyrimidinede formule II ou une 2-mercapto-4-hydroxy-6-fluoralcoxypyrimidine de formule V et le chlorodifluorométhane ou un 1,1-difluoralcène de formule III est effectuée en présence d'hydroxyde de lithium, d'hydroxyde de sodium, d'hydroxyde de potassium, d'hydroxyde de calcium, d'hydroxyde de strontium ou d'hydroxyde de baryum.

**7.** Procédé selon la revendication 6, caractérisé en ce que la réaction entre une 2-mercapto-4-halogéno-6-hydroxypyrimidinede formule II ou une 2-mercapto-4-hydroxy-6-fluoralcoxypyrimidine de formule V et le chlorodifluorométhane ou un 1,1-difluoralcène de formule III est effectuée en présence d'hydroxyde de sodium ou d'hydroxyde de potassium.

**8.** Procédé selon la revendication 1, caractérisé en ce que les bases sont mises en oeuvre à l'état de solutions aqueuses.

**9.** Procédé selon la revendication 1, caractérisé en ce que la réaction entre une 2-mercapto-4-halogéno-6-hydroxypyrimidinede formule II ou une 2-mercapto-4-hydroxy-6-fluoralcoxypyrimidine de formule V et le chlorodifluorométhane ou un 1,1-difluoralcène de formule III est effectuée en présence d'un catalyseur à transfert de phase.

**10.** Procédé selon la revendication 9, caractérisé en ce que le catalyseur à transfert de phase est mis en oeuvre en quantité de 1 à 20 mol % par rapport à la 2-mercapto-4-halogéno-6-hydroxypyrimidine de formule II ou à la 2-mercapto-4-hydroxy-6-fluoralcoxypyrimidine de formule V.

**11.** Procédé selon les revendications 9 et 10, caractérisé en ce que l'on utilise en tant que catalyseur à transfert de phase un éther en couronne ou un sel d'ammonium quaternaire.

**12.** Procédé selon les revendications 9 et 10, caractérisé en ce que l'on utilise en tant que catalyseur à transfert de phase le 18-couronne-6, le bromure de tétrabutylammonium ou le chlorure de benzyltriéthylammonium.

**13.** Procédé selon la revendication 1, caractérisé en ce que la réaction entre une 2-mercapto-4-halogéno-6-fluoralcoxypyrimidine de formule IV et un nitrite de métal alcalin ou alcalino-terreux est effectuée dans un alcane-carboxamide liquide, un alcane-carboxynitrile liquide, un lactame, un dialkylsulfoxyde ou un alcanol en C1-C4.

**14.** Procédé selon la revendication 13, caractérisé en ce que la réaction entre une 2-mercapto-4-halogéno-6-fluoralcoxypyrimidine de formule IV et un nitrite de métal alcalin ou alcalino-terreux, est effectuée dans le formamide, le N,N-diméthylformamide, le N,N-diméthylacétamide, la N-méthylpyrrolidone, le diméthylsulfoxyde, le méthanol, l'éthanol ou l'isopropanol, servant de solvant.

**15.** Procédé selon la revendication 14, caractérisé en ce que la réaction entre une 2-mercapto-4-halogéno-6-fluoralcoxypyrimidine de formule IV et un nitrite de métal alcalin ou alcalino-terreux est réalisée dans le formamide, le N,N-diméthylformamide ou le diméthylsulfoxyde, servant de solvant.

**16.** Procédé selon la revendication 15, caractérisé en ce que la réaction entre une 2-mercapto-4-halogéno-6-fluoralcoxypyrimidine de formule IV et un nitrite de métal alcalin ou alcalino-terreux est effectuée dans le N,N-diméthylformamide qui sert de solvant.

**17.** Procédé selon la revendication 1, caractérisé en ce que l'on utilise en tant que nitrite de métal alcalin ou alcalino-terreux le nitrite de sodium, le nitrite de potassium, le nitrite d'ammonium, le nitrite de magnésium, le nitrite de calcium ou le nitrite de baryum.

**18.** Procédé selon la revendication 17, caractérisé en ce que l'on utilise le nitrite de sodium ou le nitrite de potassium.

**19.** Procédé selon la revendication 1, caractérisé en de que l'on utilise de 2 à 4 mol de nitrite alcalin ou alcalino-terreux par mol de la 2-mercapto-4-halogéno-6-fluoralcoxypyrimidine de formule IV.

**20.** Procédé selon la revendication 1, caractérisé en ce que la réaction entre une 2-mercapto-4-halogéno-6-fluoralcoxypyrimidine de formule IV et un nitrite de métal alcalin ou alcalino-terreux est effectuée à une température dans l'intervalle de 50 à 200 ° C.

**21.** Procédé selon la revendication 20, caractérisé en ce que la réaction entre une 2-mercapto-4-halogéno-6-fluoralcoxypyrimidine de formule IV et un nitrite de métal alcalin ou alcalino-terreux est effectuée à une température de 130 à 160 ° C.

**22.** 2-mercapto-4-halogéno-6-fluoralcoxypyrimidines de formule IV

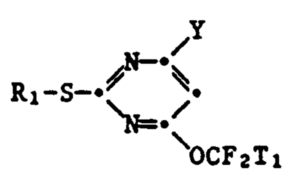

(IV)

dans laquelle $R_1$ représente un groupe alkyle en C1-C4, phényle ou benzyle, Y représente le chlore ou le brome et $T_1$ représente l'hydrogène ou un groupe $-CHX_1X_2$ dans lequel $X_1$ et $X_2$ représentent chacun, indépendamment l'un de l'autre, un groupe trifluorométhyle, le fluor, le chlore ou le brome.